# EUROPEAN PATENT APPLICATION

(11) **EP 3 741 351 A1**
(43) Date of publication of application: **25.11.2020**
(21) Application number: 20186039.2
(22) Date of filing: 16.04.2015
(51) Int. Cl.: A61K 8/24, A61K 8/34, A61K 8/60, A61K 8/73, A61Q 19/08

(54) **COMPOSITION FOR IMPROVING THE APPEARANCE OF AGEING SKIN**

(30) Priority: 17.04.2014 EP 14165164
(62) Divisional of application: 15715312.3
(71) Applicant: Merz Pharma GmbH & Co. KGaA, 60318 Frankfurt am Main (DE)
(72) Inventor: Otto, Sabine, 61138 Niederdorfelden (DE); Pooth, Rainer, 65812 Bad Soden (DE); Hagedorn, Benjamin, 60488 Frankfurt (DE)
(74) Representative: Jacobi, Markus Alexander

(57) **Abstract**

A liquid composition for improving the facial contour and shape of the human or animal face by injection, the liquid composition comprising water and at least one of the following components B, A, and C:
at least one high viscosity component B, which leads to a viscosity of the liquid composition in the range from 10 to 1500 mPas,
at least one component A, which leads to an osmotic concentration in the range from 500 to 5000 mOsm in the liquid composition,
at least one micro-particles component C, comprising micro-particles with an average particle size not larger than 0.25 mm, preferably in the range of 0.001 mm to 0.25 mm, and optionally comprising
a local anesthetic compound D.

## Description

The present invention relates to a composition for improving the appearance of ageing skin and of facial structures, such as contour and shape. It also deals with the treatment of facial ligaments with liquid compositions or pharmaceutical formulations comprising particulate, viscosity increasing or osmotic substances.

The optical appearance of the human face is strongly influenced by the skin but also by the structure of the face and in particular the facial ligaments. Ligaments are fibrous condensations which are osteo-cutaneous and musculo-cutaneous supporting structures. They often connect bones to other bones. They are also known as articular ligaments or fibrous ligaments. Ligaments are located at many parts of the human body, such as knees or elbows, but also found under the skin of the human face.

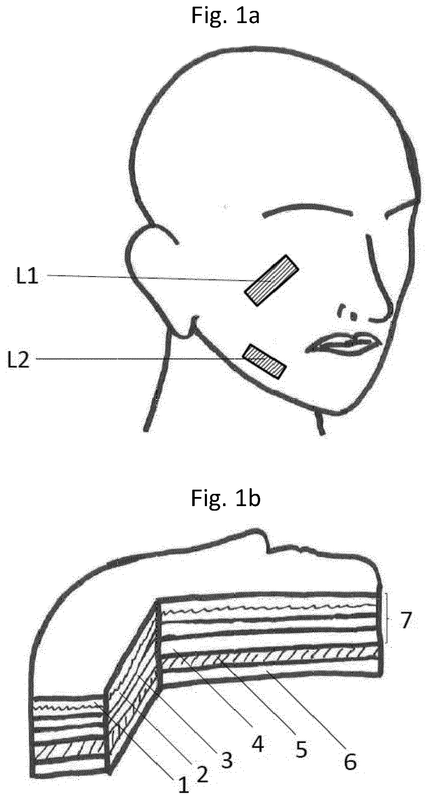

The skin of the human face in general is a multifunctional organ, including the function of protection against environmental influences. Skin is built up from several different layers (see Fig. 1b), which decrease in elasticity and resilience over time due to age-related modifications. The facial skin of a person is especially exposed to environmental influences and countenance. Therefore, there is a high need for a convenient treatment for tightening of ageing facial structures and skin.

Exemplarily, in the human cheek region, most muscles are more or less tightly but continuously connected together and with the skin by means of interwoven connective tissue, the so called superficial muscular aponeurotic system (SMAS). From there, strands run toward the skin (false retaining ligaments) and form the septa of the compartments. There are also strands of connective tissue that insert into bone, called true retaining fibers. The mid-facial ageing is described by B.C. Mendelson (Surgical anatomy of the midcheek-facial layers, Clin Plast Surg. 2008, 35, 395-404) and by A.E. Wulc (The anatomic basis of midfacial ageing, 2012, 15-28).

The morphologic changes over time occurring in the mid face and the pathogenesis of midfacial ageing based on anatomic components are discussed. Mendelson postulates that ligamentous laxity may be responsible for facial ageing.

The author proposes that the ligaments serve to stabilize the face in youth, but that continuous muscular activity in combination with intrinsic ageing changes can lead to weakness of ligamentous support in the face.

Various types of so-called "prolotherapy" are known for strengthening lax ligaments, e.g. in the knee. The mechanism of action of prolotherapy is not well understood. Proliferating agents are injected into stretched or torn ligaments of the knee, resulting over weeks in the loss of pain in the affected area. The proliferant compounds seem to lead to a localized inflammation which diminishes gradually over days. The efficiency of prolotherapy was reviewed by R.G. Schwarz (J Neurol Orthop Med Surg., 12, 1991, 220-223). Two-thirds of the patients reported a reduction of sacroiliac low back pain. Prolotherapy is applied as an injection-based treatment for chronic pain. Schwarz concluded that it is possible to induce proliferation of collagen in human ligaments of the human back by using prolotherapy. The tissue proliferating is a dense collagen and seems associated with a reduction in pain.

A. Banks (Journal of Orthopaedic Medicine, 13, 1991, No 3) summarized potential substances (irritants, particulates, osmotics, and chemotactics) for prolotherapy. The first classes of proliferant solutions, called irritants, are e.g. phenol, guaiacol and tannic acid. Particulates, such as pumice flour, are small particles of about 1 micron, which attract macrophages. Other proliferants lead to an osmotic shock. These agents act by dehydrating cells at the injection site. The chemotactics are represented by sodium morrhuate, a mixture of fatty acid salts. The chemotactic composition contains the biosynthetic precursors to certain chemotactic agents which attract inflammatory cells.

M.J. Yelland (Spine, 29, 2004, 2126-2133) described randomized controlled trials of prolotherapy injections for chronic low back pain. Prolotherapy injections alone were not found more effective than control injections alone. However, M. Scarpone (Clin J Sport Med., 18, 2008, 248-254) published a pilot study wherein the efficacy of prolotherapy for lateral epicondylosis was tested. Prolotherapy with dextrose and sodium morrhuate seemed well tolerated and decreased elbow pain.

Y. Akihiro in "Effects of hyperosmolality on the central nervous system and intracranial hemorrhage", Brain and Development, 1982, 4, 353-360, describes the injection of hyper-osmotic compositions comprising glucose, NaCl and NaHCO₃.

B. Petersen in "A glucose-dependent mechanism in jejunum inhibits gastric acid secretion: a response mediated through enteroglucagon", Scandinavian Journal of Gastroenterology, 1985, 20, 193-197, describes the influence of glucose (300-1200 mOsm/l) on secretion in the stomach.

US 2011/195925 describes a process for sterilization of hyaluronic acid compositions, which have a high osmolarity. WO 2007/077399 proposes a gel of hyaluronic acid and glycerol for dermal applications. FR-A 2938187 (2010) discloses an injectable formulation of hyaluronic acid and glycerol and lidocaine. WO 2000/37047 proposes a kit for an injectable composition of hyaluronic acid salt and lidocaine. In WO 2006/015131, facial tissue strengthening and tightening devices are described. The devices can be used by surgeons to provide uniform facial tissue planes. The possibility of applying chemical agents for facial tightening with the devices is mentioned.

The human face is covered by complexly composed skin and comprises various ligaments. Figure 1a shows a human face with mouth, nose and ears and two exemplified ligaments (L1 and L2). The different layers of facial skin are shown in Figure 1b.

The first layer is the epidermis (Figure 1b-1) which is composed of the outermost layers of cells in the skin; the thickness can vary from 0.05 mm to 1.5 mm. The second layer is the dermis (Figure 1b-2).

Main components are dermal cells (fibroplasts, histiocytes and mast cells), dermal fibers (collagen fibers and reticulin fibers), and dermal matrix. Dependent on the location, the thickness of the dermis varies strongly.

The third layer is the subcutaneous tissue (Figure 1b-3) containing mainly lax conjunctive tissue. It is followed by the fourth layer, the musculoaponeurotic (Figure 1 b-4), relating to muscular and aponeurotic tissue. It is attached by retinacular cutis fibers within the subcutaneous layer to the dermis and the epiderm is.

These four layers (Figure 1b-7) form a functional unit which is mobile because loose areolar tissue and retaining ligaments (Figure 1b-5) permits gliding movement of the composite scalp over the fixed sixth layer, periosteum and deep fascia (Figure 1b-6).

Transferred to the mid cheek (see Figure 1a) the musculo-aponeurotic is described as superficial muscular aponeurotic system (SMAS). The SMAS contains the intrinsic muscles of the cheek, which have a limited attachment to the underlaying facial skeleton and a more extensive attachment to the soft tissue which they move.

Optical changes of the human face, which originate from ageing, mostly occur in the midface. These optical changes may occur due to gravity, fat atrophy, and deterioration in the condition and appearance of the skin. The facial ligaments serve as stabilizer in the youth face. Continuous muscular activity in combination with intrinsic ageing changes lead to weakness of ligamentous support.

Since many years, surgery is a known treatment of aged facial skin, see B.C. Mendelson (Surgical anatomy of the midcheek, Clin Plast Surg.). However, surgery is uncomfortable, painful und unsightly. There is a high need for a better treatment of ageing facial structures and facial skin, with reduced or no side effects. This improvement can be applied by a minimally invasive method.

The present invention relates to a liquid composition for improving the facial contour and shape of the human or animal face by injection, the liquid composition comprising water and at least one of the following components B, A and C:
a) a high viscosity component B, which leads to a viscosity of the liquid composition in the range from 10 to 1500 mPas, in particular from 200 to 1000 mPas,
b) a component A, which leads to an osmotic concentration in the range from 500 to 5000 mOsm, in particular from 1200 to 4000 mOsm in the liquid composition,
c) a micro-particles component C, comprising micro-particles with an average particle size not larger than 0.25 mm, preferably in the range of 0.001 mm to 0.25 mm, in particular in the range of 0.001 mm to 0.1 mm, and optionally comprising
d) a local anesthetic compound D.

Often, the composition is administered for improving the ligamentous laxity of the face via injection either into the or close to the ligaments of the face (e.g. in an amount of 0.1 to 1.5 ml).

Often, two of these components are combined in the liquid composition, such as A+B, A+C or B+C. In one embodiment, three different of these components are combined in the liquid composition, such as A+B+C, A+C+D or A+B+C or B+C+D. In one embodiment, four different components A+B+C+D are combined in the liquid composition. Preferably, compositions comprise at least one component B.

The invention further relates to a liquid composition for improving the facial contour and shape of the human face, wherein the composition is administered for improving the ligamentous laxity of the face, and where the injection occurs into the or close to the ligaments of the face.

The invention further relates to a liquid composition which comprises as component A at least one saccharide or C₃-C₆-polyol, in particular selected from the group comprising glucose, mannose, saccharose, fructose, mannitol and glycerol. According to the invention, component A may also be selected from further compounds defined in the description. The osmolarity (mOsm) can be determined by known methods (e.g. at 25°C).

The invention further relates to a liquid composition which comprises as component B at least one compound selected from the group comprising hyaluronic acid, hydroxypropyl methylcellulose (HPMC) and carboxymethylcellulose (CMC). According to the invention, component B may also be selected from further compounds (or mixtures of compounds) defined in the description. The viscosity (mPas) can be determined by known methods, e.g. by Oswald viscosimeter or glass capillary viscometer (at 25°C).

The invention further relates to a liquid composition which comprises as component C a component having an average particle size in the range of 0.001 mm to 0.05 mm, which component is selected from the group comprising polylactic acid, polycaprolactam, hydroxyapatite and pumicite. According to the invention, component C may also be selected from further compounds defined in the description. The average particle size can be determined by known methods, such as microscopic analysis, light scattering and others.

The invention further relates to a liquid composition wherein the composition additionally comprises at least one physiologically compatible (acceptable) salt S, in particular selected from the group comprising sodium chloride, sodium bicarbonate and zinc sulfate.

The invention further relates to a liquid composition which comprises at least 50 % (w/w) of water, at least two of the components A, B and C and optionally also comprises a local anesthetic compound D. For some uses, it is preferred to have the compositions not containing a compound D.

The invention further relates to a liquid composition which comprises:
60 to 89 % (w/w) of water,
10 to 35 % (w/w) of component A, and
1 to 25 % (w/w) of component B,
and optionally comprises a micro-particles component C and/or a local anesthetic compound D. Depending on the type of component A, the amount can also be smaller then 10 % (w/w), if this leads to an osmotic concentration in the range from 500 to 5000 mOsm. The component B should be different from component A.

The invention further relates to a liquid composition which comprises:
60 to 89 % (w/w) of water,
10 to 35 % (w/w) of a saccharide component A, leading to an osmotic concentration of 1200 to 4000 mOsm in the liquid composition, and
1 to 25 % (w/w) of high viscosity component B, leading to a viscosity of the liquid composition in the range of 200 to 1000 mPas, and optionally comprising up to 15 % (w/w) of a micro-particles component C with micro-particles with an average particle size in the range of 0.001 mm to 0.1 mm.

The invention further relates to a liquid composition which is for injection administration via blunt cannula in an area nearby the ligament, or via sharp cannula or sharp needle into the ligament. The choice of injection device can be important and can be related to the properties of the liquid composition, e.g. to the viscosity.

The invention further relates to a process for the preparation of a liquid composition for injection according to any of the claims 1 to 10, comprising the step of mixing at least one of the components A, B and C, and optionally component D, with water. In one embodiment, all three types of components A, B and C are combined in the liquid composition.

The invention further relates to a process for the preparation of a liquid composition for injection according to claim 11, comprising the step of mixing at least two of the components A, B and C, and optionally component D, with water.

The invention further relates to a method for optical improving the facial contour and shape of the human face comprising the steps of:
a) cleaning specific skin areas above the ligaments of the face;
b) injection of an aqueous liquid composition into or nearby specific ligaments of the face,
   where the liquid composition contains:
   at least one saccharide or C₃-C₆-polyol component A, which leads to an osmotic concentration of 1200 to 4000 mOsm in the liquid composition,
   and optionally
   a high viscosity component B, which leads to a viscosity of the liquid composition in the range of 200 to 1000 mPas, and optionally a micro-particles component C, comprising micro-particles with an average particle size in the range of 0.001 mm to 0.1 mm;
c) and optionally repeating the injection step b) several times.

This method for optical improving the facial contour and shape of the human face can be a cosmetic method or a therapeutic method.

The invention further relates to a method for optical improving, wherein the volume of the liquid composition applied for each injection step is in the range from 0.01 to 1.2 ml, often from 0.02 to 0.9 ml.

The invention further relates to a method for optical improving, wherein in step several injections of the aqueous liquid composition are administered at specific areas of the face into or nearby specific ligaments of the face, and this procedure is repeated from 1 to 10 times, often from 2 to 8 times.

Component A of the composition of present invention may be selected from any compound or mixture of compounds which leads to an osmotic concentration in the range from 500 to 5000 mOsm.

Component A in particular may be selected from the group comprising mono-, di-, and oligosaccharides. Monosaccharides may be selected from the group comprising dioses (C2), trioses (C3), tetroses (C4), pentoses (C5), hexoses (C6), and heptoses (C7). The monosaccharides may occur in various stereoisomeric forms represented by saccharides such as D- and L-glyceraldehyde, D- and L-erythrose, D- and L-threose, D- and L-erythrulose, D- and L-ribose, D- and L-arabinose, D- and L-xylose, D- and L-lyxose, D- and L-desoxyribose, D- and L-ribulose, D- and L-xylulose, D- and L-allose, D- and L-altrose, D- and L-glucose, D- and L-mannose, D- and L-gulose, D- and L-idose, D- and L-galaktose, D- and L-talose, D- and L-glucuronic acid, D- and L-galacturonic acid, N-acetyl-D-glucosamine, N-acetyl-L-glucosamine, D- and L-glucosamine, N-acetyl-D-galactosamine, N-acetyl-L-galactosamine, D- and L-fucose, D- and L-rhamnose, D- and L-chinovose, D- and L-fructose, or a mixture thereof. Disaccharides may be selected from the group comprising sucrose, lactulose, lactose, maltose, trehalose, cellobiose, kojibiose, nigerose, isomaltose, β,β-trehalose, α,β-trehalose, sophorose, laminaribiose, gentiobiose, turanose, maltulose, palatinose, gentiobiulose, mannobiose, melibiose, melibiulose, rutinose, rutinulose, xylobiose, or a mixture thereof.

In a preferred embodiment, the saccharide of component A is selected from the group comprising D- and L-ribose, D- and L-glucose, sucrose, lactulose, lactose, maltose or a mixture of two or more thereof.

Further, component A may be selected from one or several C₃-C₆-polyols. Polyols may be linear or branched. Preferably C₃-C₆-polyols are selected from the group comprising glycerol, xylitol, pentaerythritol, mannitol, or a mixture thereof. Particular preferable C₃-C₆-polyols are selected from the group comprising glycerol and mannitol.

Further, component A may be selected from the group comprising polyether, exemplarily polyethylene glycol (PEG) of different molecular weights, polytetramethylene glycol, polytetramethylene ether glycol, or a mixture thereof. PEG may in principal be any molecular weight PEG, preferably however PEG 6000 or similar products are used.

In another preferred embodiment of present invention component A is selected from saline solutions, exemplarily a sodium chloride solution. For these saline components A, the amount in the composition can be smaller than 10 % (w/w), e.g. 5 %, as long as this leads to an osmotic concentration in the range from 500 to 5000 mOsm.

Further, component A may be a combination or mixture of at least one saccharide and at least one C₃-C₆-polyol. Preferably, a combination or mixture comprises one type of saccharide and one type of polyol. A combination or mixture comprising one type of saccharide and one type of polyol may vary in the weight ratio from 1:10 to 10:1 saccharide: polyol.

The liquid compositions of the present invention often comprise component A from 0 to 35 % (w/w), preferably from 10 to 35 % (w/w), particular preferably from 15 to 25 % (w/w). Component A may lead to an osmolality in the liquid composition in a range of 500 to 5000 mOsm, preferably 1200 to 4000 mOsm, and particular preferably 2000 to 3000 mOsm. Osmole is a unit of measurement that defines the number of moles of solute that contribute to the osmotic pressure of a solution (at room temperature, 25 °C).

The liquid composition of the present invention may optionally comprise a high viscosity component B. This component B leads to an increased viscosity of the composition at room temperature. By using the adequate amount of component B, the viscosity of the composition can be increased and the concentration of the proliferant compound (or compounds) remains for a prolonged time at the injected area. Suitable compounds to increase the viscosity, which are physiologically acceptable, may e.g. be:
hyaluronic acid, hydroxypropyl methylcellulose (HPMC), carboxymethylcellulose (CMC), polyvinyl alcohol (PVA) or polyvinyl pyrrolidone(PVP).

Preferably component C is selected from the group comprising hyaluronic acid, hydroxypropyl methylcellulose (HPMC), and carboxymethylcellulose (CMC).

The liquid compositions of the present invention often comprise at least one component B in an amount of from 0 to 25 % (w/w), preferably from 1 to 25 % (w/w), particular preferably from 5 to 25 % (w/w).

The preferred viscosity may be in the range of 10 mPas to 1500 mPas, preferably 200 to 1000 mPas, and particular preferably 300 to 800 mPas at 25 °C. Viscosity is due to the friction between neighboring particles in a fluid that are moving at different velocities. It can be measured by classical viscometers or rheometers, such as glass capillary viscometer. Typical viscosities for some known compounds at 25 °C are (for comparing): 0.9 mPas (water), 0.2 mPas (n-pentane), 0.6 mPas (benzene), 2.3 (acetic acid 80%), and approximately 100 mPas (engine oil).

The composition of the present invention may comprise at least one micro-particles component C. Particles according to component C of the present invention may be bio-degradable polymers.

Component C in particular is selected from the bio-degradable polymer group comprising:
polylactic acid, polycaprolactone, cellulose ester, polyhydroxyalkanoate, starch derivatives, poly(butylene adipate-co-terephthalate), and poly-(butylene succinate).

Particles of component C of the present invention may additionally be selected from the group comprising hydroxyapatite and pumicite.

Preferably component C is selected from the group comprising polylactic acid, polycaprolactam, hydroxyapatite and pumicite.

Compositions of the present invention often comprise component C from 0 to 15 % (w/w), preferably from 1 to 10 % (w/w), particular preferably from 5 to 10 % (w/w).

The micro-particles preferably have an average particle size not larger than 0.25 mm, preferably in the range of 0.001 mm to 0.25 mm, preferably in the range of 0.001 mm to 0.1 mm, and particular preferably in the range of 0.001 mm to 0.05 mm. The particles may have different forms (e.g. spherical) and different types of particle-size distributions. The particle size can be determined by filters.

Component D, a local anesthetic, may optionally be added to the composition of the present invention. Appropriate local anesthetic compounds may be selected from the group comprising esters, amines, combination thereof, and naturally derived local anesthetic.

The ester anesthetics may preferably be selected from the group comprising:
benzocaine, chloroprocaine, cocaine, cyclomethycaine, dimethocaine/larocaine, piperocaine, propoxycaine, procaine/novocaine, and proparacaine, tetracaine/amethocaine.

The amine class of local anesthetics may preferably be selected from the group comprising: articaine, bupivacaine, hcinchocaine/dibucaine, etidocaine, levobupivacaine, lidocaine/lignocaine, mepivacaine, prilocaine, and ropivacaine, trimecaine.

The class of naturally derived local anesthetics may preferably be selected from the group comprising: saxitoxin, neosaxitoxin, tetrodotoxin, menthol and eugenol.

Compositions of the present invention often comprise component D from 0 to 5 % (w/w), preferably from 0.1 to 3 % (w/w), often from 0.5 to 2 % (w/w).

Furthermore, in a different embodiment of the invention, the composition optionally may comprise additional physiologically compatible salts, e.g. from 0 to 25 % (w/w). Compatible salts may preferably be selected from the group comprising sodium chloride, sodium bicarbonate, and zinc sulfate, or a mixture of two or more thereof. The osmolality of the composition of present invention may be influenced by addition of said salts.

The process for the preparation of a composition for the injection of present invention comprises the step of solving or suspending at least one component A, B, or C in an excipient, in particular water (sterile). Optionally, component D, and additional physiologically compatible salts may be added to the composition.

In a preferred embodiment, the process for the preparation of a composition for the injection of present invention comprises the step of mixing at least one component A, at least one component B, and optionally further components C and D.

In another preferred embodiment, the process for the preparation of a composition for the injection of present invention comprises the step of mixing at least one component A, at least one component B, at least one component C, and optionally further component D.

In another preferred embodiment, the process for the preparation of a composition for the injection of present invention comprises the step of mixing at least one component A, at least one component B, at least one component C, at least one physiologically compatible salt, and optionally further component D.

In another preferred embodiment, the process for the preparation of a composition for the injection of present invention comprises the step of mixing at least one component A, at least one component B, at least one component C, and optionally further component D, wherein the excipient is water.

The composition comprising water in a range of 60 to 89 %(w/w) may e.g. contain component A in the range of 10 to 35 % (w/w), component B in the range of 1 to 25 % (w/w) and component C in a range of 0 to 15 % (w/w), and component D in a range of 0 to 5 %(w/w).

The composition comprising water in a range 60 to 89 %(w/w) may e.g. contain component A in the range of 10 to 35 % (w/w), component B in the range of 1 to 25 % (w/w) and component C in a range of 1 to 10 % (w/w), and component D in a range of 0 to 5 %(w/w).

The composition comprising water in 60 to 89 %(w/w) may contain component A in the range of 10 to 35 % (w/w), component B in the range of 1 to 25 % (w/w) and component C in a range of 1 to 10 % (w/w), and a physiologically compatible salt in a range of 0 to 25 % (w/w), and component D in a range of 0 to 5 %(w/w).

The prolongation of duration of action of the proliferant compound(s) may be increased in the variation of the excipients. The viscosity of the composition for injection should be in the range of 10 mPas to 1500 mPas at 25 °C. Thus, the viscosity may be increased in changing from water to further lipid excipients (or emulsions thereof). The formulations of the composition of the invention may exist in various forms.

Hence, they may be a solution, an emulsion or microemulsion of the type oil-in-water (O/W), a multiple emulsion, for example of the type water-in-oil-in-water (W/O/W). The liquid composition of present invention may preferably be formulated in water.

The compositions of present invention may comprise a solution and/or suspension comprising the proliferating agent.

Examples of excipients, besides water, are hydrocarbons such as soft or liquid paraffin, an oil of natural origin such as almond, corn, arachis, castor, sunflower, lini crudum, sesame, soya or olive oil or derivatives thereof; or a fatty acid and/or ester such as steric, palmitic or oleic acid. Also medium-chain fatty acid triglycerides and isopropyl myristate are of interest.

Compositions of present invention comprise an excipient from 60 to 89 % (w/w), often from 60 to 80 % (w/w). The preferred excipient is water.

Compositions according to the present invention may further comprise a suspending agent, a wetting agent, one or more of a preservative, a buffer, an antioxidant, an anti-inflammatory agent, a bactericide, a substance for preventing foaming and a surface active agent. The ingredients for the emulsion, suspension, or solution are selected from ingredients known in the art. Particular ingredients may function as two or more of these agents simultaneously, e.g. behave like a preservative and a buffer.

Suitable surface active agent or emulsifier such as an anionic, cationic, or nonionic surfactant may be represented by sorbitan ester, polysorbate, Cremophor EL, polysorbate 20, polysorbate 80, macrogol-20-glycerolmonostearat, and/or mixtures thereof. Suspending agents selected from the group comprising natural gums, cellulose derivatives, and other ingredients such as lanolin.

The composition of the present invention can be injected into, or near the ligament of a human or animal to be treated. The composition of the present invention may further be injected with the needle toughing bone.

The process or method of treatment by injection is not limited to a single injection at a specific area. The treatment of ligaments via injection of a composition according to present invention may also be performed at several ligaments in parallel or in sequence.

Blunt cannulas may be too blunt to penetrate ligaments. The injection via a blunt cannula occurs in an area close to the ligament. Trained persons injecting the composition will feel resistance of the ligament and inject aforesaid composition in implied area. The composition should be injected at least 0.5 mm from treating ligament, preferably 0.2 mm.

Sharp cannulas or sharp needles can penetrate into ligaments. The injection via sharp cannula or sharp needle occurs into the ligament. Trained persons injecting the composition will feel resistance of the ligament, insert into it and inject aforesaid composition into the ligament. Suitable ligaments may be selected from the group of false retaining ligaments, comprising masseter-cutaneous retinacula, bucco-maxillar retinacula (buccal part), and platysma-auricular retinacula. Further, ligaments may be selected from the group of true retaining ligaments, comprising orbital retinacula, zygomatic retinacula (McGregor's patch), bucco-maxillar retinacula (maxillar part), mandibular retinacula, platysma-mandibular ligament, and submental ligament.

Preferably, the composition of present invention may be applied to the bucco-maxillar retinacula (L1) and/or the platysma-mandibular (L2) depicted in Figure 1a, in particular it can be administered to the Zygomatic ligaments (McGregor's patch).

The composition of the present invention may be filled up in flacons, ampoules, vessels, or prefilled syringes. In one embodiment, the composition is located in prefilled syringes (ready to use syringe, RTUS), which can markedly improve the safety of use of injectable preparations. Sterilization of the RTUS is performed via heat, radiation, chemical, or filtration sterilization. Preferably the sterilization may occur via heat or UV-irradiation.

The method for optical improving the facial contour and shape of the human face comprises the administration of a composition of present invention. This process comprises the injection into, or near the ligament to be treated. In a first step, the skin areas above the ligaments of the face should be cleaned. In a second step, an aqueous liquid composition into or nearby specific ligaments of the face containing at least one of the component A, B, or C and optionally component D is injected. This injection step may optionally be repeated several times, preferably 0 to 20 times, particular preferably 1 to 10 times. The composition of the present invention may further be injected with the needle.

The volume of composition applied for each injection is in the range of 0.01 to 1.5 ml, preferably 0.01 to 1.2 ml. Particular preferably the volume of injection is in the range of 0.02 to 0.9 ml.

The process of injection, wherein several injections of the aqueous composition are administered at specific areas of the face, may be repeated from 1 to 20 times, preferably from 1 to 10 times. However, a single treatment may be sufficient. Intervals of injection may range from 5 days to 10 months, preferably 1 day to 9 months. However, a single treatment may be sufficient.

### Example 1 (Preparation of the liquid compositions)

1a) 30 % (w/w) glucose
   5 % (w/w) hyaluronic acid (25 °C)
   2 % (w/w) hydroxyapatite
   63 % (w/w) water.
1b) 30 % (w/w) glucose
   2 % (w/w) hydroxyapatite
   0.5 % (w/w) lidocaine
   67.5 % (w/w) water
1c) 20 % (w/w) glucose
   10 % (w/w) hyaluronic acid (25 °C)
   11 70 % (w/w) water
1d) 21 % (w/w) hyaluronic acid (25 °C)
   0.5% (w/w) lidocaine 78.5 % (w/w) water
1e) 21 % (w/w) hyaluronic acid (25 °C)
   5 % (w/w) sodium chloride,
   74 % (w/w) water.
1f) 30 % (w/w) glucose
   5 % (w/w) hyaluronic acid (25 °C)
   2 % (w/w) hydroxyapatite
   0.5 % (w/w) lidocaine
   62,5% (w/w) water

### Example 2 (Testing of the compositions)

The compositions according to experiments 1a to 1f can be administered by injection to old rats, aged 24 months.

| Group | Treatment | Number of Animals | Time Point | # MCLs for Biomechanics | # MCLs for Collagen Typing |
|---|---|---|---|---|---|
| 1 | Vehicle Control | 8 | 30 days | 8 | 8 |
| 2 | Ex. 1c | 8 | 30 days | 8 | 8 |
| 3 | glucose | 8 | 30 days | 8 | 8 |
| | Totals: | 24 | | 24 | 24 |

ANIMALS: 24 Sprague-Dawley Rats (~24 months of age)
HOUSING: Group Housed.
TEST ARTICLE: Compositions of examples 1a to 1e
DOSE ROUTE/FREQUENCY: Each rat receives bilateral injections into the Medial Collateral Ligament (MCL) at 1, 7, 14, 21, and 28 days.
DOSE PREPARATION: Prepared daily on dosing days.
CAGESIDE OBSERVATIONS: Twice daily (mortality/moribundity)
BODY WEIGHTS: Day 1, 7, 14, 21 & 30.
NECROPSY: All animals are euthanized and the right and left hind limbs are disarticulated at the hip joint. Extraneous tissue are dissected away to carefully expose the femur-MCL-tibia complex. The biomechanical properties of the MCL are then be evaluated using an Instron Mechanical Test System.
IMMUNOHISTOCHEMISTRY (n=48): The left MCL for all animals are placed in 10% formalin for IHC staining of Collagen Type I and III. The MCLs from each rat are be imbedded in paraffin and sectioned. One slide will be stained for Type I and a second for Type III. The percent of Collagen (positive stain area/total tissue area) for Type I and III are then be reported.
BIOMECHANICS (n=24): The right MCL for all animals undergo destructive testing. The femur-MCL-tibia complex are mounted into a custom holder. A pre-lead of (0.1N) is applied and the MCL preconditioned (cyclical loading to 1% strain for 10 cycles). The ligament initial length and cross-sectional area is determined. The ligament is then be pulled to failure at 10% per second.

### Example 3 (Testing of the compositions)

The compositions according to experiments 1a to 1f can be administered via sharp needle into e.g. the human zygomatic ligaments (McGregor's patch) in an explorative clinical trial. Different product concentrations and treatment regimens are to be used for dose-finding and dose-regimen (trials might be conducted in separate settings).

As examples, the liquid composition 1a or 1f is administered via sharp needle into (or alternatively 2 mm next to) the human facial ligament (0.5 ml, twice daily for three days) for improving the laxity of the face.

## Claims

1. Liquid composition for improving the facial contour and shape of the human or animal face by injection, the liquid composition comprising water and at least one of the following components B, A and C:
a) a high viscosity component B, which leads to a viscosity of the liquid composition in the range from 10 to 1500 mPas, in particular from 200 to 1000 mPas,
b) a component A, which leads to an osmotic concentration in the range from 500 to 5000 mOsm, in particular from 1200 to 4000 mOsm in the liquid composition,
c) a micro-particles component C, comprising micro-particles with an average particle size not larger than 0.25 mm, preferably in the range of 0.001 mm to 0.25 mm, in particular in the range of 0.001 mm to 0.1 mm, and optionally comprising
d) a local anesthetic compound D.

2. Liquid composition according to claim 1 for improving the facial contour and shape of the human face, wherein the composition is administered for improving the ligamentous laxity of the face, and where the injection occurs into the or close to the ligaments of the face.

3. Liquid composition according to claim 1 or 2, which comprises as component A at least one saccharide or C₃-C₆-polyol, in particular selected from the group comprising glucose, mannose, saccharose, fructose, mannitol and glycerol.

4. Liquid composition according to any of claims 1 to 3, which comprises as component B at least one compound selected from the group comprising hyaluronic acid, hydroxypropyl methylcellulose (HPMC) and carboxymethylcellulose (CMC).

5. Liquid composition according to any of claims 1 to 4, which comprises as component C a component having an average particle size in the range of 0.001 mm to 0.05 mm, which component is selected from the group comprising polylactic acid, polycaprolactam, hydroxyapatite and pumicite.

6. Liquid composition according to any of claims 1 to 5, wherein the composition additionally comprises at least one physiologically compatible salt S, in particular selected from the group comprising sodium chloride, sodium bicarbonate and zinc sulfate.

7. Liquid composition according to any of the claims 1 to 6, which comprises at least 50 % (w/w) of water, at least two of the components B, A and C and optionally also comprises a local anesthetic compound D.

8. Liquid composition according to any of the claims 1 to 7, which comprises:
60 to 89 % (w/w) of water,
10 to 35 % (w/w) of component A, and
1 to 25 % (w/w) of component B,
and optionally comprises a micro-particles component C and/or a local anesthetic compound D.

9. Liquid composition according to any of the claims 1 to 8, which comprises:
60 to 89 % (w/w) of water,
10 to 35 % (w/w) of a saccharide component A, leading to an osmotic concentration of 1200 to 4000 mOsm in the liquid composition, and
1 to 25 % (w/w) of high viscosity component B, leading to a viscosity of the liquid composition in the range of 200 to 1000 mPas, and optionally
comprising up to 15 % (w/w) of a micro-particles component C with micro-particles with an average particle size in the range of 0.001 mm to 0.1 mm.

10. Liquid composition according to any of claims 1 to 9, which is for injection administration via blunt cannula in an area nearby the ligament, or via sharp cannula or sharp needle into the ligament.

11. Process for the preparation of a liquid composition for injection according to any of the claims 1 to 10, comprising the step of mixing at least one of the components A, B and C, and optionally component D, with water.

12. Process for the preparation of a liquid composition for injection according to claim 11, comprising the step of mixing at least two of the components A, B and C, and optionally component D, with water.

13. Method for optical improving the facial contour and shape of the human face comprising the steps of:
a) cleaning specific skin areas above the ligaments of the face;
b) injection of an aqueous liquid composition into or nearby specific ligaments of the face,
where the liquid composition contains:
at least one saccharide or C₃-C₆-polyol component A, which leads to an osmotic concentration of 1200 to 4000 mOsm in the liquid composition,
and optionally
a high viscosity component B, which leads to a viscosity of the liquid composition in the range of 200 to 1000 mPas, and optionally a micro-particles component C, comprising micro-particles with an average particle size in the range of 0.001 mm to 0.1 mm;
c) and optionally repeating the injection step b) several times.

14. Method for optical improving according to claim 13, wherein the volume of the liquid composition applied for each injection step b) is in the range of 0.01 to 1.2 ml.

15. Method for optical improving according to claim 13 or 14, wherein in step b) several injections of the aqueous liquid composition are administered at specific areas of the face into or nearby specific ligaments of the face, and this procedure is repeated from 1 to 10 times.
